(19) Europäisches Patentamt
European Patent Office
Office européen des brevets

(11) Numéro de publication: **0 418 140 A1**

(12) **DEMANDE DE BREVET EUROPEEN**

(21) Numéro de dépôt: **90402491.6**

(22) Date de dépôt: **11.09.90**

(51) Int. Cl.5: **C09K 19/20**, C09K 19/30, C07C 69/75

(30) Priorité: **14.09.89 FR 8912026**

(43) Date de publication de la demande: **20.03.91 Bulletin 91/12**

(84) Etats contractants désignés: **CH DE FR GB LI NL**

(71) Demandeur: **THOMSON-CSF**
**51, Esplanade du Général de Gaulle**
**F-92800 Puteaux(FR)**

(72) Inventeur: **Baillon-Moussel, Christine**
**THOMSON-CSF, SCPI, Cédex 67**
**F-92045 Paris la Défense(FR)**
Inventeur: **Broussoux, Dominique**
**THOMSON-CSF, SCPI, Cédex 67**
**F-92045 Paris la Défense(FR)**
Inventeur: **Le Barny, Pierre**
**THOMSON-CSF, SCPI, Cédex 67**
**F-92045 Paris la Défense(FR)**
Inventeur: **Soyer, Françoise**
**THOMSON-CSF, SCPI, Cédex 67**
**F-92045 Paris la Défense(FR)**

(74) Mandataire: **Guérin, Michel et al**
**THOMSON-CSF SCPI 51, Esplanade du Général de Gaulle**
**F-92045 PARIS LA DEFENSE CEDEX 67(FR)**

(54) **Cristaux liquides chiraux dérivés du biphényle.**

(57) L'invention concerne des cristaux liquides chiraux dérivés du biphényle et répondant aux formules générales suivantes :

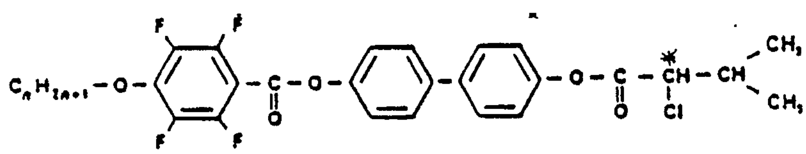

avec 1 ≦ n ≦ 14.

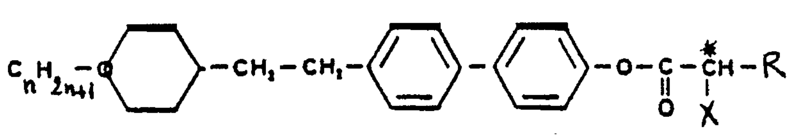

avec 1 ≦ n ≦ 14
X = Cl ou Br

EP 0 418 140 A1

R est 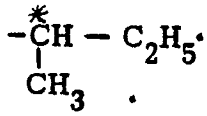 ou 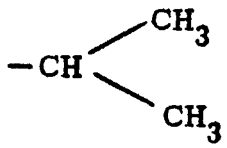 .

## CRISTAUX LIQUIDES CHIRAUX DERIVES DU BIPHENYLE

La présente invention concerne des cristaux liquides chiraux dérivés du biphényle et utilisables soit comme dopants, soit en mélange.

La communication joue un rôle privilégié dans notre civilisation engendrant des besoins technologiques nouveaux comme celui de la visualisation de données et d'informations sous forme d'images en couleurs. Le tube cathodique bien qu'actuellement très performant et peu coûteux ne semble pas être capable de satisfaire tous les besoins de la visualisation du futur et en particulier l'obtention d'écrans ayant une surface active supérieure au mètre carré. C'est dans ce contexte technique que l'on voit apparaître de nouvelles technologies d'écrans de grandes dimensions tels que les écrans à plasma, les écrans électroluminescents et les écrans à cristaux liquides.

Jusqu'à présent, seuls les cristaux liquides nématiques ont été développés et ce principalement dans des systèmes de visualisation utilisant l'effet nématique en hélice. Mais les dispositifs de ce type ont un angle de vue limité et un temps de réponse élevé, de l'ordre de la dizaine de millisecondes, ce qui limite leur emploi.

En 1980, N.A. CLARK et S. LAGERWALL (Appl. Phys. Lett. Vol. 36, n° 89, 1980) ont montré que des temps de réponse très courts, de l'ordre de la dizaine de microsecondes pouvaient être obtenus avec des cristaux liquides en mettant à profit les propriétés ferroélectriques de la phase smectique C*. Ces dernières apparaissent lorsque la phase $S_C$* est introduite dans une cellule dont l'épaisseur est inférieure au pas du matériau. Depuis cette date, un effort de recherche important est fait pour obtenir des matériaux $S_C$* bien adaptés aux besoins de l'affichage.

En première approximation, le temps de réponse $\tau$ d'un mélange $S_C$* est donné par la relation suivante :

$\tau = \eta / Ps\ E$

où $\eta$ représente la viscosité rotationnelle du cristal liquide, Ps sa polarisation spontanée et E le champ électrique appliqué. Les matériaux les plus performants sont ceux qui présentent une forte polarisation spontanée, associée à une faible viscosité rotationnelle.

Deux voies sont possibles pour obtenir des mélanges $S_C$* :

- soit optimiser un mélange de cristaux liquides présentant tous une phase $S_C$*
- soit introduire un ou plusieurs dopants chiraux dans une matrice $S_C$.

C'est cette seconde voie qui paraît la plus prometteuse et la plus fexible. Dans ce cas, les propriétés finales des mélanges dépendent d'une part des propriétés de la matrice $S_C$ et d'autre part, des propriétés des dopants chiraux.

La matrice $S_C$ apporte : la succession des phases, le domaine d'existence en température de ces phases, la faible viscosité, l'anisotropie diélectrique et la biréfringence.

Les dopants chiraux apportent la chiralité et la polarisation.

Le dopant doit par ailleurs ne pas modifier les températures de transition de la matrice et ne pas augmenter la viscosité du mélange final.

Afin de satisfaire toutes ces conditions, la présente invention propose deux nouvelles familles de cristaux liquides chiraux dérivées du biphényle, utilisables comme dopant d'une matrice $S_C$ et, lorsque les composés selon l'invention présentent une phase $S_C$*, utilisables pour réaliser des mélanges $S_C$* avec d'autres cristaux liquides présentant une phase $S_C$*.

L'invention a donc pour objet un cristal liquide chiral dérivé du biphényle, caractérisé en ce qu'il répond à la formule générale suivante :

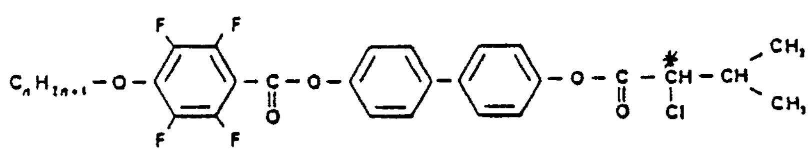

avec $1 \leq n \leq 14$.

L'invention a aussi pour objet un cristal liquide chiral dérivé du biphényle, caractérisé en ce qu'il répond à la formule générale suivante :

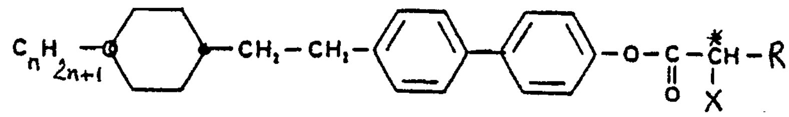

avec $1 \leq n \leq 14$
X = Cl ou Br

R est 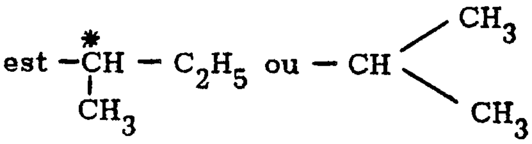 ou

L'invention a encore pour objet un mélange cristal liquide présentant une phase smectique C chirale, caractérisé en ce qu'il est constitué d'une composition présentant une phase smectique C dopée par un cristal liquide selon l'une des revendications 1 ou 2 afin de conférer au mélange ladite phase smectique C chirale.

La présente invention sera mieux comprise et d'autres avantages apparaîtront à la lecture de la description qui va suivre, donnée à titre non limitatif, et grâce aux figures annexées parmi lesquelles :

- les figures 1 et 2 représentent des formules chimiques de composés selon l'invention,
- les figures 3 et 4 représentent des schémas de synthèse de composés selon l'invention,
- la figure 5 est un diagramme représentant la polarisation de mélanges selon l'invention en fonction de la température,
- la figure 6 est un diagramme représentant la polarisation d'un composé selon l'invention en fonction de la température,
- la figure 7 est un diagramme représentant la polarisation de mélanges selon l'invention en fonction de la température,
- les figures 8 et 9 sont des diagrammes représentant l'angle de tilt en fonction de la température pour des mélanges selon l'invention,
- les figures 10 et 11 sont des diagrammes représentant le temps de réponse en fonction de la température pour des mélanges selon l'invention.

La figure 1 représente la formule chimique d'une première famille de composés selon l'invention. Pour cette famille, le nombre n vaut : $1 \leq n \leq 14$.

La figure 2 représente la formule chimique d'une deuxième famille de composés selon l'invention. Pour cette famille, le nombre n vaut : $1 \leq n \leq 14$, X est Cl ou Br et R est

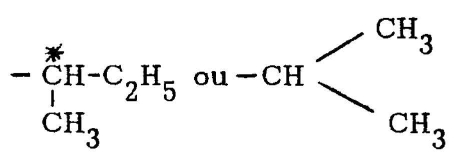 ou

Ces deux familles présentent les avantages suivants :

- la présence de 3 cycles dans la structure chimique des deux familles conduit à l'existence de mésophases sur une large gamme de température,
- l'existence d'un atome d'halogène (Cl ou Br) attaché directement au carbone asymétrique, et la proximité de ce carbone asymétrique du coeur mésogène concourent à l'obtention d'une polarisation élevée,
- enfin, l'utilisation d'un noyau benzénique perfluoré (première famille) ou d'un radical cyclohexane éthyl trans (deuxième famille) conduisent à des composés de faible viscosité comparés aux dérivés benzoïques correspondants.

**SYNTHESE DES COMPOSES SELON L'INVENTION**

- Synthèse des composés de la première famille.

La synthèse des dérivés de cette famille se fait en 5 étapes à partir de l'acide perfluorobenzoïque et de la valine selon le schéma réactionnel décrit figure 3.

- Synthèse des composés de la deuxième famille.

La synthèse des composés de la deuxième famille se fait en 8 étapes à partir de l'acide 4 alkylbenzoïque et de la valine ou de l'isoleucine selon le schéma réactionnel de la figure 4.

Certaines étapes des schémas de synthèse représentés aux figures 3 et 4 ne posent aucun problème particulier à l'homme du métier. On va décrire ci-dessous plus en détail les quelques étapes qui pourraient poser problème.

. Synthèse de l'acide 2 chloro 3 méthyl pentanoïque.

(Produit I de la figure 4 quand R = $C_2H_5$- $CH(CH_3)$ et X = Cl).

Dans un erlenmeyer de 250 ml muni d'une agitation magnétique, on dissout 13,1 g (0,1 mole) d'isoleucine dans 64 ml d'acide chlorhydrique 6N. On refroidit le mélange à 0°C et on additionne goutte à goutte une solution de 8,3 g (0,12 mole) de nitrite de sodium dans le minimum d'eau. On observe la formation de vapeurs nitreuses.

On laisse agiter à 0° C pendant 2 heures. On ajoute alors de l'urée pour détruire l'excès d'acide nitreux. On extrait à l'éther. La phase éthérée est séchée sur $MgSO_4$, filtrée et évaporée à sec. On obtient 7 g de produit brut jaune. Le produit brut est distillé sous vide. La température d'ébullition sous 1 mm de mercure $E_1$ vaut 98° C. Le rendement est de 25 % .

. Synthèse de l'acide 2 chloro 3 méthyl butanoïque.

(Produit II de la figure 3).

Cet acide se synthétise de la même manière que l'acide 2 chloro 3 méthyl pentanoïque.

Le rendement est de 20,5 % .

La température d'ébullition sous 10 mm de mercure $E_{10}$ vaut 98° C.

. Synthèse du trans 4 diazoacétyl 4′ heptyl cyclohexane

(Produit III de la figure 4 avec n = 7).

Dans un erlenmeyer de 250 ml muni d'une agitation magnétique, maintenu à -10° C, on introduit la solution éthérée de diazométhane obtenu à partir de 10,3 g (0,1 mole) de nitrosométhylurée, 5 ml de triéthylamine et 8,1 g de chlorure de 4 heptylcyclohexanecarbonyle (0,033 mole) en solution dans 50 ml d'éther. On maintient la solution à 0° C pendant 1 heure, puis on laisse revenir à la température ambiante et on continue d'agiter pendant 15 heures. On coule dans l'eau et on extrait à l'éther. La phase organique est séchée sur $MgSO_4$ filtrée et évaporée à sec. On obtient 8 g de solide orange. Le rendement est de 96 % . Le point de fusion du produit obtenu est de 71° C.

. Préparation de l'acide 4 heptyltranscyclohexyl acétique.

(Produit IV de la figure 4 avec n = 7).

Dans un erlenmeyer de 500 ml muni d'une agitation magnétique, on introduit 140 ml d'eau, 3,5 g (0,034

mole) de carbonate de sodium et 3,5 g de thiosulfate de sodium pentahydraté (0,014 mole). On chauffe la solution à reflux et on ajoute goutte à goutte une solution de 14 g de trans 4 diazoacétyl 4′ heptylcyclohexane (0,056 mole) dans 75 ml de dioxane. On chauffe à reflux pendant 36 heures. La solution est ensuite filtrée sur terre d'infusoire. Le filtrat est acidifié avec l'acide nitrique puis on effectue une extraction à l'éther. La phase organique est lavée à l'eau, séchée sur $MgSO_4$, filtrée et évaporée à sec. On obtient 11,4 g de produit brut qui, recristallisé dans l'éthanol, donne 3,6 g de solide blanc. Le rendement est de 26,7 %. Le point de fusion du produit obtenu est de 54° C.

. Préparation du 4 méthoxy 4′ heptyltranscyclohexylacéthyl biphényle.

(Produit V de la figure 4 avec n = 7).

Dans un erlenmeyer de 250 ml muni d'une agitation magnétique, on introduit 45 ml de chlorure de méthylène, 9 g (0,049 mole) de 4 méthoxybiphényle et 11,6 g (0,045 mole) de chlorure de 4 heptyltranscyclohexylacétyle. On refroidit le milieu réactionnel à 0° C et on ajoute par petites quantités 6,6 g de chlorure d'aluminium (0,048 mole). On laisse revenir à température ambiante et on continue d'agiter pendant 15 heures.

Le milieu réactionnel est versé dans HCl concentré à 25 % . On extrait à l'éther. On obtient 18 g de produit brut qui est recristallisé dans le toluène. On obtient 5,9 g de solide. Le rendement est de 32 % .

. Synthèse du 4 hydroxy 4′ heptyltranscyclohexyléthyl biphényle.

(Produit VI de la figure 4 avec n = 7).

Dans un erlenmeyer de 50 ml muni d'une agitation magnétique, on introduit 1,8 g de potasse (0,032 mole), 5,6 ml d'hydrazine hydratée, 30 ml de diéthylène glycol et 5,9 g (0,0145 mole) de 4 méhoxy 4′ heptyltranscyclohexylacéthyl biphényle. On chauffe à 110° C pendant 3 heures, on distille ensuite l'azéotrope eau hydrazine.

On refroidit la solution, on ajoute de l'eau et on extrait à l'éther. Le produit brut est recristallisé dans l'éthanol. Le point de fusion du produit obtenu est de 176° C.

**PROPRIETES DES CORPS SYNTHESISES.**

Les composés selon l'invention ont été étudiés par analyse enthalpique différentielle, microscopie optique et diffraction des rayons X. L'ensemble de ces observations a permis de déterminer la structure des mésophases présentées par les composés selon l'invention. Les résultats sont rassemblés dans les tableaux 1 et 2 placés en fin de description, le tableau 1 concernant des composés de la première famille (figure 1) et le tableau 2 des composés de la seconde famille (figure 2). La lettre K désigne la phase cristalline, $S_C$* une phase smectique C chirale, N* une phase cholestérique, $S_B$ une phase smectique B, $S_A$ une phase smectique A et L la phase liquide. Les températures de transition de phase sont exprimées en °C.

Les composés selon l'invention ont été introduits à titre de dopants dans un mélange M présentant une phase $S_C$ (mélange Merck référencé ZLI 3234 B) et possédant la succession de phases suivante :
$S_C$ 76 $S_A$ 80 N 96 L.

Les propriétés suivantes ont été mesurées :
- températures de transition du mélange final,
- polarisation en fonction de la température,
- angle de tilt en fonction de la température,
- mesure du temps de réponse.

Pour obtenir un mélange homogène après incorporation du dopant on a procédé de la manière suivante. Après pesée du dopant et du mélange M dans un même récipient, on réalise une solution homogène par ajout d'un solvant purifié à bas point d'ébullition ($CH_2Cl_2$ ou $CHCl_3$). Cette solution est ensuite filtrée sur filtre Millipore 1 μm en environnement dépoussiéré. Enfin, le solvant est éliminé par

évaporation lente sous courant d'azote à 40 ° C, puis sous vide.

Tous les dopants selon l'invention sont solubles à une concentration de 10 % en poids dans le mélange.

Les températures de transition des mélanges dopés (à raison de 10 % en poids de dopant dans le mélange final obtenu) ont été déterminées par analyse enthalpique différentielle et par microscopie optique. Les résultats sont rassemblés dans le tableau 3 placé en fin de description.

Le signe Ø indique que la mésophase a été observée au microscope sur quelques dixièmes de degré mais la température de transition n'a pu être déterminée par analyse enthalpique différentielle.

La mesure de polarisation, du temps de réponse et de l'angle de tilt nécessite la réalisation de cellules de mesures. Les cellules sont faites à partir de lames de verre rectangulaires (2 x 3 cm) de 1 mm d'épaisseur, recouvertes d'ITO (oxyde mixte d'indium et d'étain) non recuit. La fabrication des cellules se fait en 5 étapes.

On définit d'abord sur chaque lame de verre une bande conductrice de 1 cm de largeur au moyen d'une technique de microlithographie utilisant la résine positive Shipley AZ 1350. Puis on enduit ces lames d'un film d'alcool polyvynylique (Rhodoviol 4/125) par dépôt à la tournette d'une solution aqueuse à 3 % . Après séchage à 110 ° C pendant une heure, le film de PVA est frotté au moyen d'une machine de frottement (par exemple de la marque Asulab) afin d'induire une orientation planaire non dégénérée. Les cellules sont ensuite scellées au moyen d'une colle araldite. Les contacts entre un fil électrique et une lame sont réalisés à l'aide de points de colles à l'argent. Enfin le matériau est introduit par capillarité.

La mesure de la polarisation à été faite en utilisant un signal triangulaire. Le champ d'excitation utilisé est de 15 V/$\mu$m. Les mesures ont été faites à 100 Hz sauf aux basses températures où la fréquence à été diminuée jusqu'à 1 Hz. Les résultats sont rassemblés dans les figures 5 à 7.

La figure 5 représente les courbes de polarisation P en fonction de la température T pour la première série de mélanges du tableau 3 : la courbe 1 pour le dopant $A_4$ (cellule de 2,4 $\mu$m d'épaisseur), la courbe 2 pour le dopant $A_5$ (cellule de 2,4 $\mu$m d'épaisseur), la courbe 3 pour le dopant $A_6$ (cellule de 1,8 $\mu$m d'épaisseur) et la courbe 4 pour le dopant $A_9$ (cellule de 2 $\mu$m d'épaisseur).

La figure 6 représente la polarisation P en fonction de la température T pour le composé $A_6$ pur.

La figure 7 représente la polarisation P en fonction de la température T pour la deuxième série de mélanges du tableau 3 : la courbe 5 pour le dopant $B_7$ (cellule de 2,3 $\mu$m d'épaisseur), la courbe 6 pour le dopant $D_7$ (cellule de 1,8 $\mu$m d'épaisseur) et la courbe 7 pour le dopant $C_7$ (cellule de 2,2 $\mu$m d'épaisseur).

La mesure de l'angle de tilt est faite en utilisant une méthode électrooptique. Un champ continu (+E) est appliqué aux bornes de la cellule qui est observée au microscope entre polariseur et analyseur croisés. Les dipôles s'alignent dans le sens du champ et on détermine la position d'extinction. Ensuite le sens du champ est inversé (-E), ce qui inverse le sens des dipôles. Les molécules ont alors tourné d'un angle 2 $\theta$ . Il faut tourner l'échantillon d'un angle 2 $\theta$ pour retrouver une position d'extinction. Le sens dans lequel il faut faire tourner l'échantillon pour retrouver une position d'extinction fournit également le signe de la polarisation. Le champ électrique appliqué est de 15 V/$\mu$m.

La figure 8 représente l'angle de tilt $\theta$ en fonction de la température T pour le mélange du tableau 3 contenant le dopant $A_5$ (cellule de 2,4 $\mu$m d'épaisseur). La figure 9 représente l'angle de tilt $\theta$ en fonction de la température T pour la deuxième série de mélange du tableau 3 : la courbe 8 pour le dopant $B_7$, la courbe 9 pour le dopant $C_7$ et la courbe 10 pour le dopant $D_7$.

Le temps de réponse électrique des matériaux à été mesuré en appliquant une tension en forme de créneaux aux bornes d'une cellule de mesure, correspondant à un champ électrique de 15 V/$\mu$m.

La figure 10 représente le temps de réponse $\tau$ en fonction de la température T pour des mélanges de la première série du tableau 3 : la courbe 11 pour le dopant $A_4$ (cellule de 13,9 $\mu$m d'épaisseur), la courbe 12 pour le dopant $A_6$ (cellule de 14,7 $\mu$m d'épaisseur) et la courbe 13 pour le dopant $A_9$ (cellule de 12,9 $\mu$m d'épaisseur).

La figure 11 représente le temps de réponse $\tau$ en fonction de la température T pour des mélanges de la deuxième série du tableau 3 : la courbe 14 pour le dopant $B_7$ (cellule de 17,5 $\mu$m d'épaisseur) et la courbe 15 pour le dopant $D_7$ (cellule de 18,7 $\mu$m d'épaisseur).

TABLEAU I

| Réf. | n | K | | $S_C^*$ | N* | | L |
|---|---|---|---|---|---|---|---|
| $A_4$ | 4 | . | | 96,5 | . | 145,5 | . |
| $A_5$ | 5 | . | 91,5 | (.58) | . | 132 | . |
| $A_6$ | 6 | . | 77 | (.66,5) | . | 135,5 | . |
| $A_9$ | 9 | . | 87 | (.81) | . | 124 | . |

| Réf. | n | X | R | K | | $S_B$ | $S_A$ | | L |
|---|---|---|---|---|---|---|---|---|---|
| $B_7$ | 7 | Cl | $-\overset{*}{C}H(CH_3)-C_2H_5$ | . | 82,5 | . | 116,5 | | . |
| $C_7$ | 7 | Br | $-\overset{*}{C}H(CH_3)-C_2H_5$ | . | 110 | . | 116,5 . | 120,5 | . |
| $D_7$ | 7 | Cl | $-CH(CH_3)-CH_3$ | . | 67 | . | 139 | | . |

TABLEAU II

TABLEAU III

| Dopant | $S_C$* | | $S_A$ | | N* | | L |
|---|---|---|---|---|---|---|---|
| $A_4$ | . | 73,1 | . | (0) | . | 98,5 | . |
| $A_5$ | . | 74,1 | . | (0) | . | 98,8 | . |
| $A_6$ | . | 73,7 | . | (0) | . | 97,8 | . |
| $A_9$ | . | 72,6 | . | (0) | . | 97,8 | . |
| $B_7$ | . | 69,5 | . | 87,5 | . | 98,9 | . |
| $C_7$ | . | 68,8 | . | 85,5 | . | 97,5 | . |
| $D_7$ | . | 67,5 | . | 87,3 | . | 98,0 | . |

## Revendications

1 - Cristal liquide chiral dérivé du biphényle, caractérisé en ce qu'il répond à la formule générale suivante :

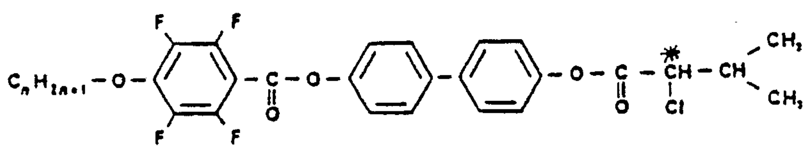

avec $1 \leqq n \leqq 14$.

2 - Cristal liquide chiral dérivé du biphényle, caractérisé en ce qu'il répond à la formule générale suivante :

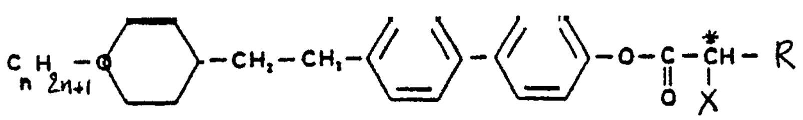

avec $1 \leqq n \leqq 14$
X = Cl ou Br

R est 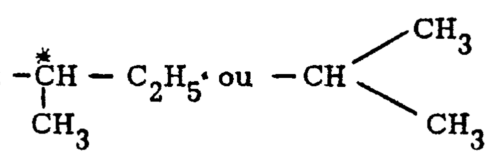 ou

3 - Cristal liquide présentant une phase smectique C chirale, caractérisé en ce qu'il est constitué d'une composition présentant une phase smectique C dopée par un cristal liquide selon l'une des revendications 1 ou 2 afin de conférer au mélange ladite phase smectique C chirale.

FIG. 1

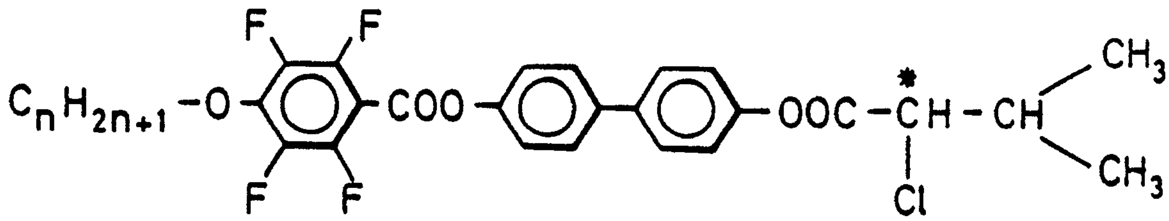

FIG. 2

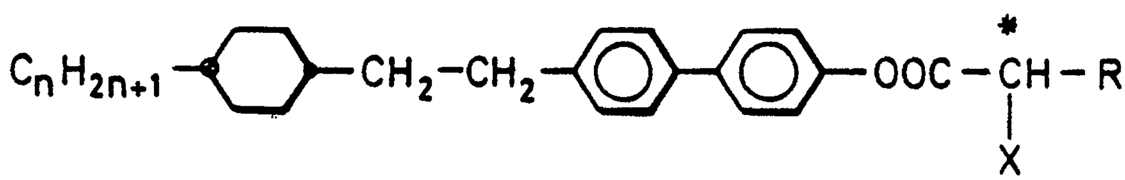

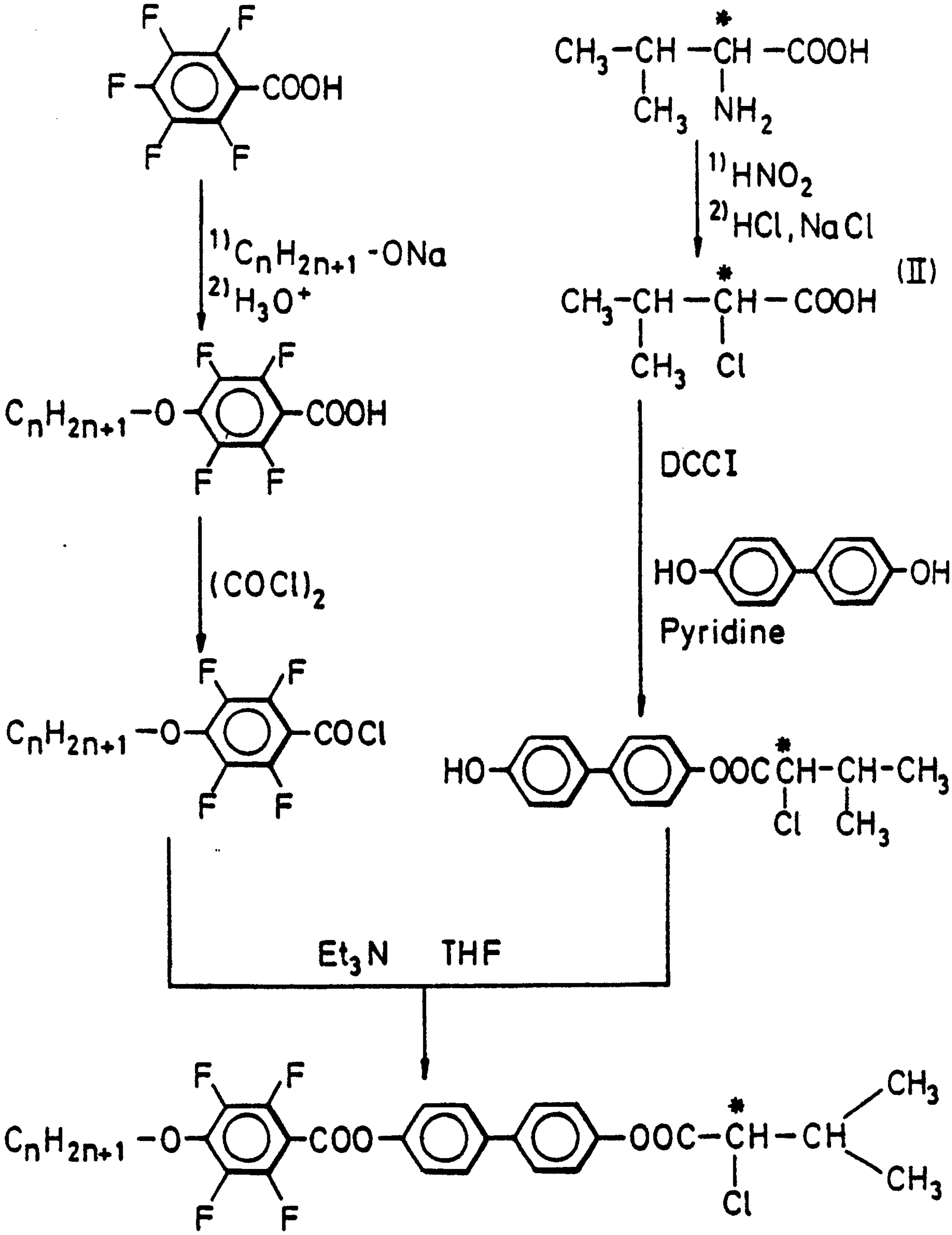
1) $C_nH_{2n+1}$-ONa
2) $H_3O^+$
1) $HNO_2$
2) HCl, NaCl
(II)
$(COCl)_2$
DCCI
Pyridine
$Et_3N$ THF

FIG.3

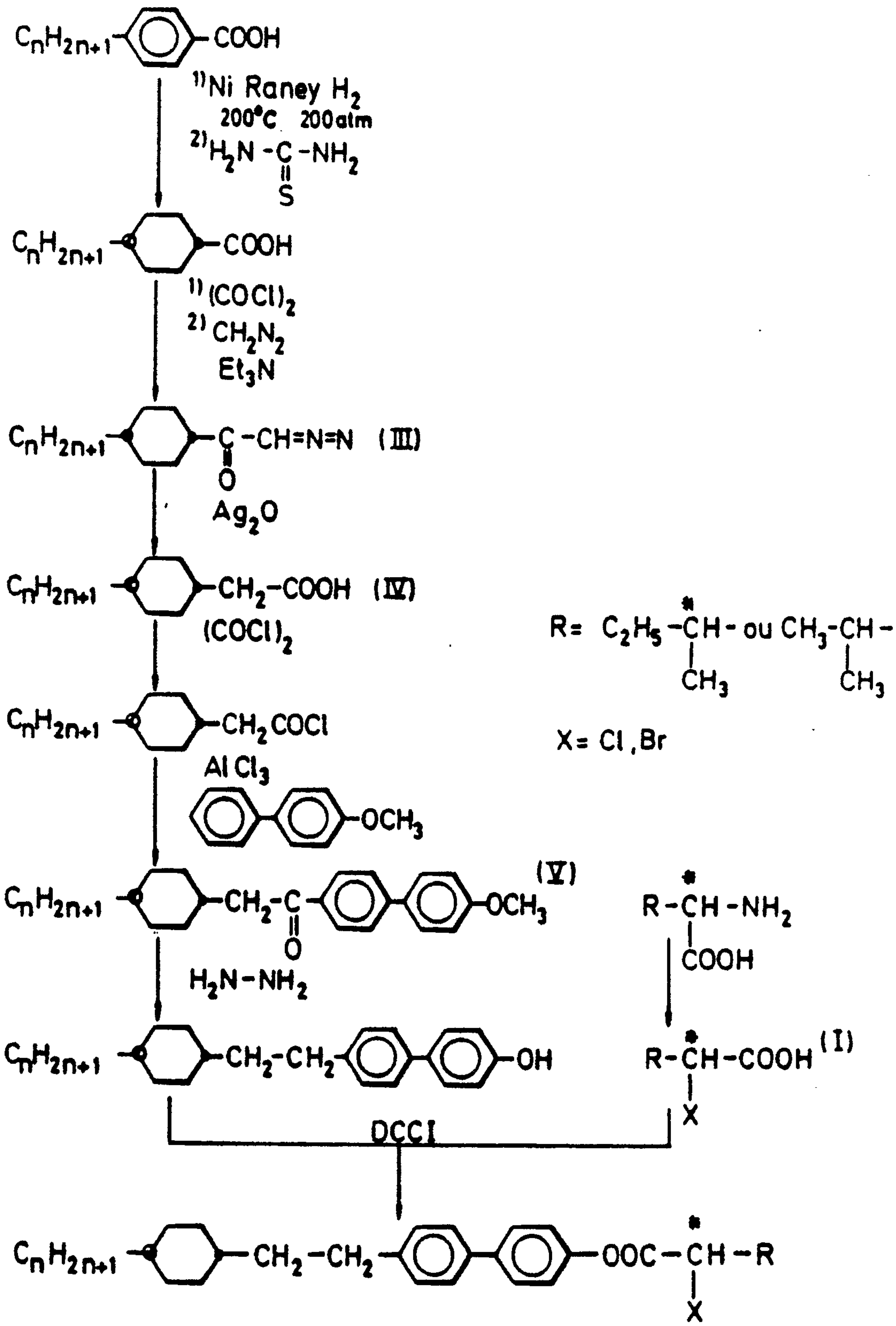
1) Ni Raney H2
200°C 200atm
2) H2N-C-NH2
S
1) (COCl)2
2) CH2N2
Et3N
(III)
Ag2O
(IV)
(COCl)2
Al Cl3
(V)
H2N-NH2
(I)
DCCI
R= C2H5-CH- ou CH3-CH-
CH3 CH3
X= Cl, Br

FIG.4

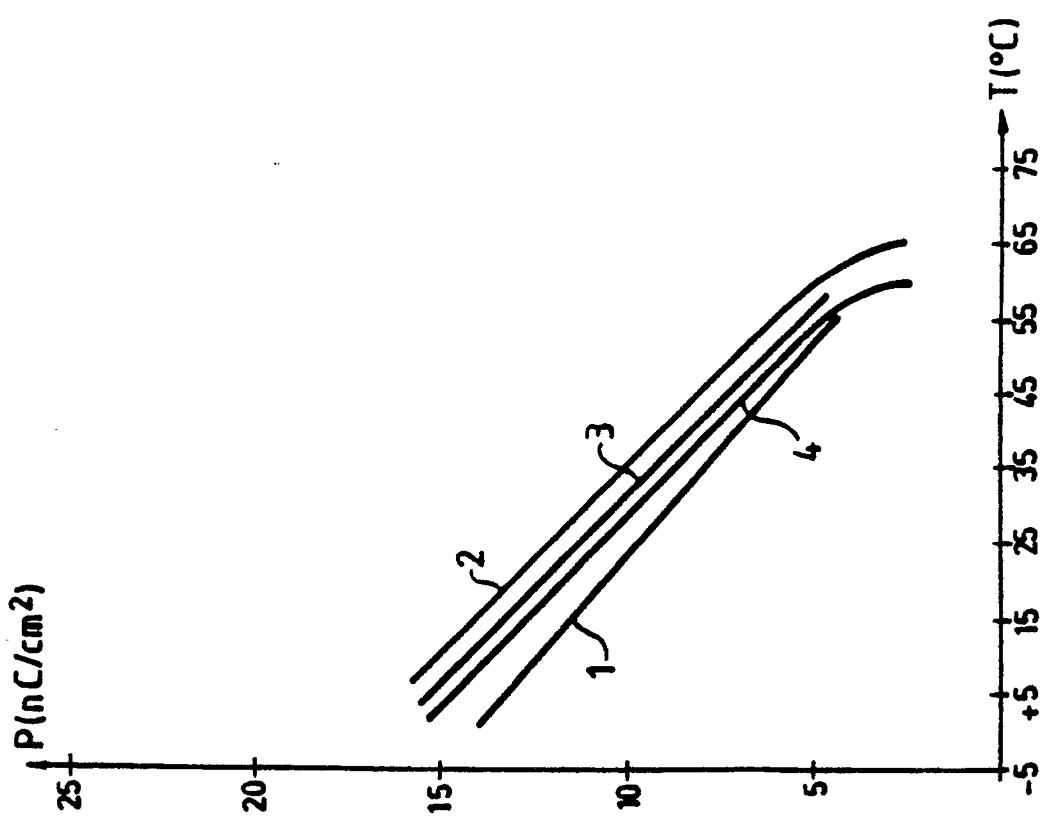
P (nC/cm2)
25
20
15
10
5
-5
+5
15
25
35
45
55
65
75
T (°C)
1
2
3
4

FIG. 5

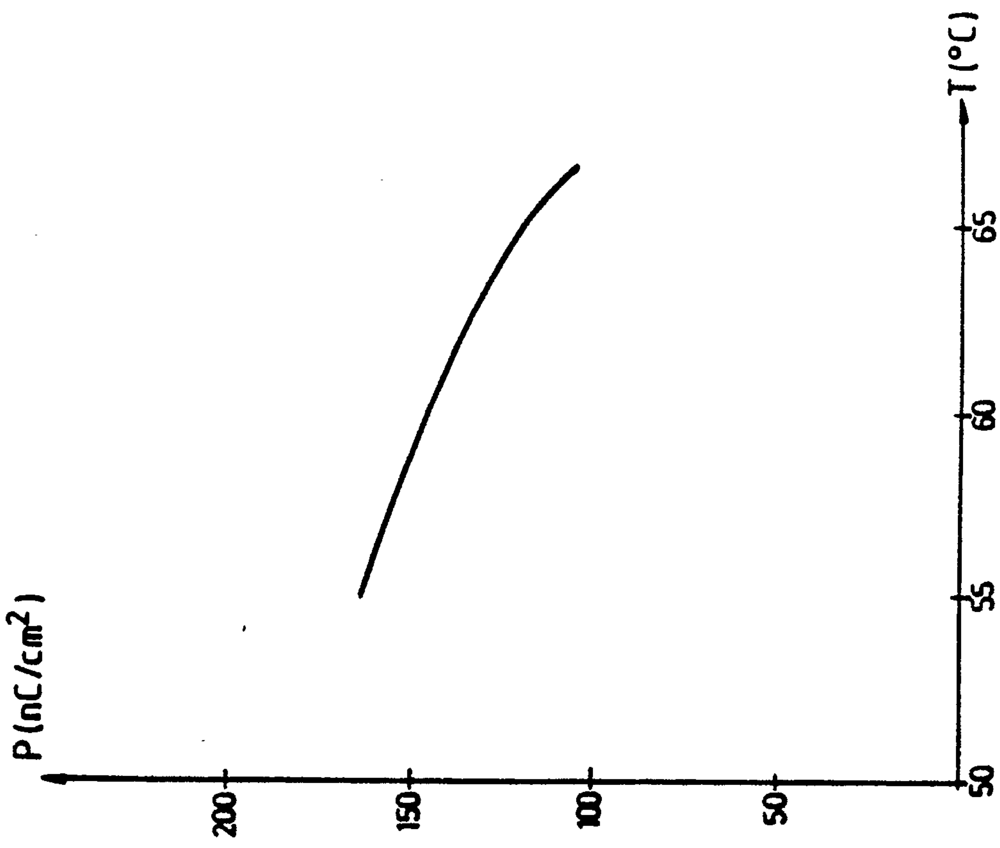
P (nC/cm2)
200
150
100
50
50
55
60
65
T (°C)

FIG. 6

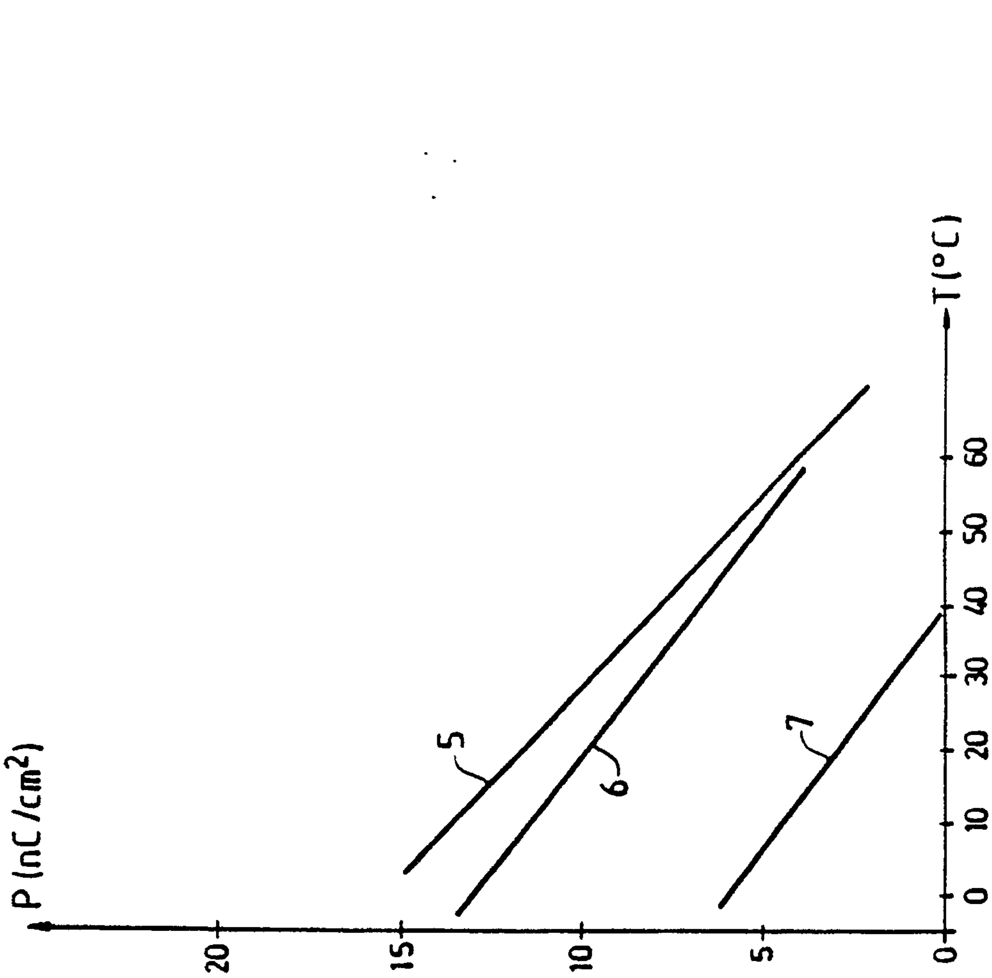
P (nC/cm$^2$)
20
15
10
5
0
0
10
20
30
40
50
60
T (°C)
5
6
7

FIG. 7

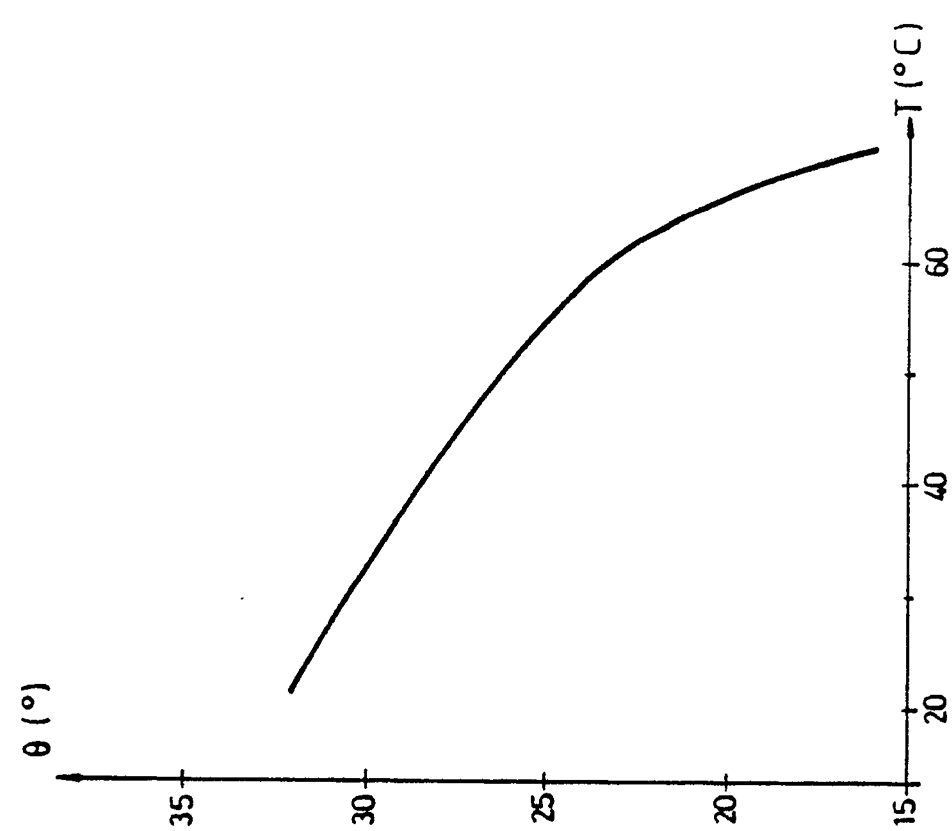
θ (°)
35
30
25
20
15
20
40
60
T (°C)

FIG. 8

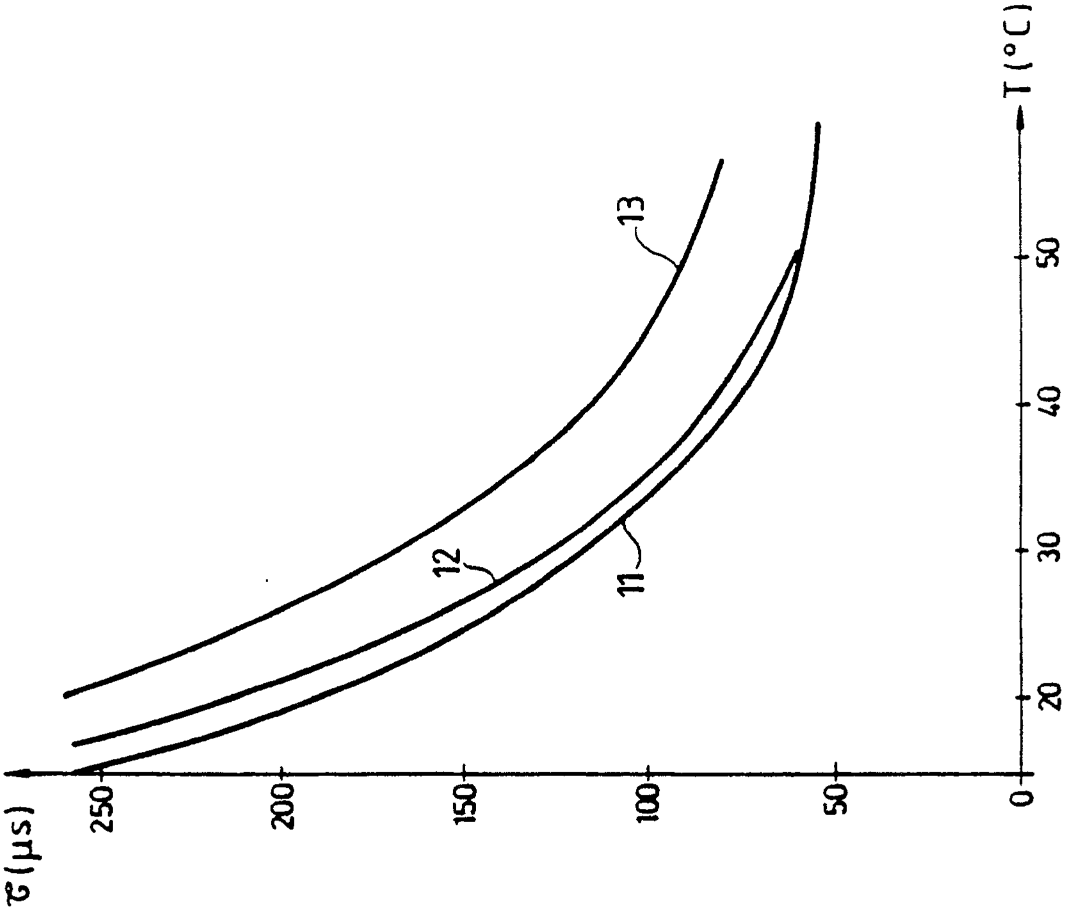
τ(μs)
250
200
150
100
50
0
20
30
40
50
T (°C)
11
12
13

FIG.10

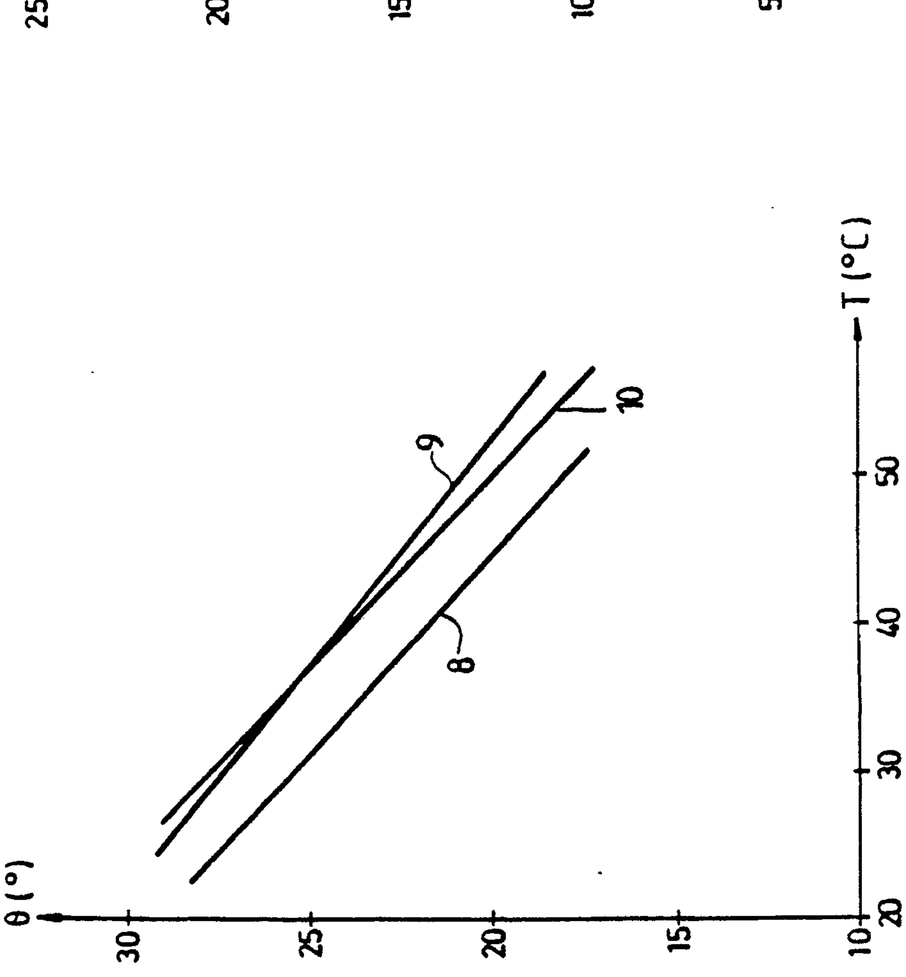
θ (°)
30
25
20
15
10
20
30
40
50
T (°C)
8
9
10

FIG.9

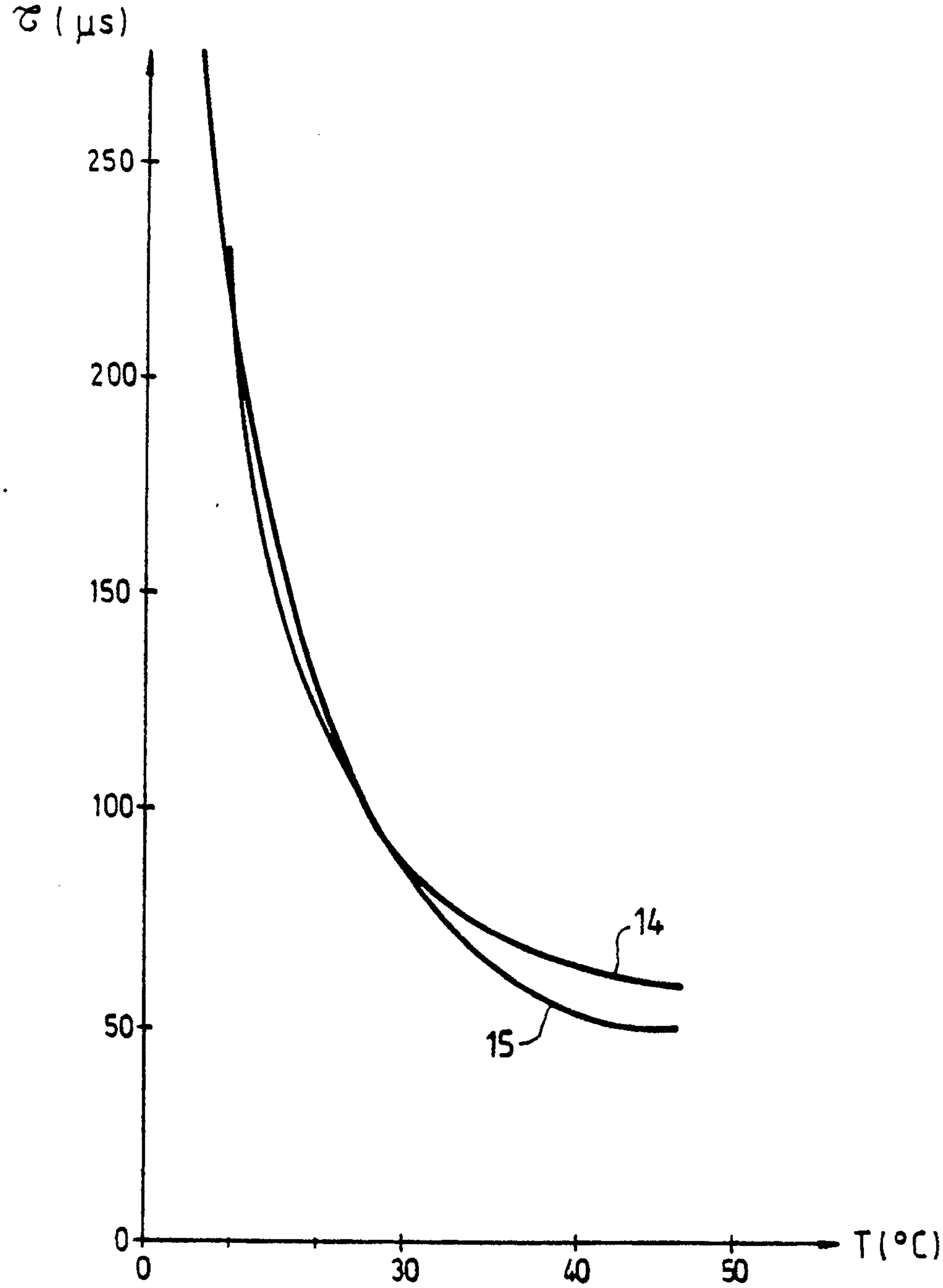


FIG.11

Office européen des brevets

# RAPPORT DE RECHERCHE EUROPEENNE

Numero de la demande

EP 90 40 2491

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | EP-A-0 360 683 (THOMSON)<br>* Page 2, lignes 41-60; page 3, lignes 1-5; page 4, lignes 12-20; page 7, lignes 61-62; revendications 1,4 *<br>--- | 1,3 | C 09 K 19/20<br>C 09 K 19/30<br>C 07 C 69/75 |
| Y | EP-A-0 011 002 (THOMSON)<br>* Page 1, lignes 13-26; page 2, lignes 1-4; revendications 1-8 *<br>--- | 1 | |
| Y | EP-A-0 191 600 (AJINOMOTO)<br>* Exemples 22,27; revendication 1 *<br>--- | 1 | |
| A | DE-A-3 410 733 (MERCK)<br>* Revendication 1; page 4; page 5, lignes 1-5 *<br>----- | 2 | |

DOMAINES TECHNIQUES RECHERCHES (Int. Cl.5)

C 09 K 19/00

Le présent rapport a été établi pour toutes les revendications

| Lieu de la recherche | Date d'achèvement de la recherche | Examinateur |
|---|---|---|
| LA HAYE | 15-10-1990 | BOULON A.F.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)